# EUROPEAN PATENT APPLICATION

(11) **EP 1 547 941 A1**
(43) Date of publication of application: **29.06.2005**
(21) Application number: 03753976.4
(22) Date of filing: 01.10.2003
(51) Int. Cl.: B65D 85/18, A61F 5/44, A61F 13/496

(54) **DISPOSABLE DIAPER PACKAGE**

(30) Priority: 02.10.2002 JP 2002289755
(71) Applicant: UNI-CHARM CO., LTD., Kawanoe-shi Ehime 799-0111 (JP)
(72) Inventor: OTSUBO, Toshifumi, Tech. Center, UNI-CHARM CO. LTD, Mitoyo-gun, Kagawa 769-1602 (JP)
(74) Representative: Staeger - Sperling
(86) International application number: PCT/JP2003/012606
(87) International publication number: WO 2004/031053

(57) **Abstract**

Here is disclosed a package of disposable diapers 1A in which a plurality of pull-on disposable diapers 10 are arranged within a bag 2 in close contact one with another in a back-and-forth direction of the diapers 10 each having waist-lateral portions 18 folded inward between themselves together with lateral portions 13c of a core 13 in front and rear waist regions 14, 15 of the diaper 10 toward a longitudinal center line 25 bisecting a width dimension defined between the waist-lateral portions 18. Of these diapers 10, intermediate portions 28 between the waist-lateral portions 18 are in close contact one with another so that the diapers 10 can be compactly packed within the bag 2.

## Description

### TECHNICAL FIELD OF THE INVENTION

The present invention relates to a package of disposable diapers and more specifically, to a package of a plurality of pull-on disposable diapers arranged within a flexible bag.

### BACKGROUND ART OF THE INVENTION

Japanese Patent Application Publication No. 1999-104177A and Japanese Patent Application Publication No. 1999-155904A disclose a pull-on disposable diaper defining front and rear waist regions opposed to each other and a crotch region extending between these waist regions, on one hand, and comprising a liquid-pervious topsheet, a liquid-impervious backsheet and a liquid-absorbent core interposed between these sheets so as to form a waist-hole and a pair of leg-holes.

The pull-on disposable diapers as disclosed in the above-cited Publications are adapted to be folded in the crotch region so that the respective inner surfaces of the front and rear waist regions may come in contact with each other to be flattened together. A set of about 16 to 48 individual diapers each flatly folded in this manner is orderly and closely packed into the package and sold per such package. For example, when 16 diapers are packed in the package, 8 diapers are arranged so that these diapers may have front and rear surfaces successively in contact one with another to form a first row extending in back-and-forth direction between opposite side walls of the package and may be laid in side-by-side relationship with the first row or laid on the first row. In each row, each pair of diapers adjacent to each other in the back-and-forth direction, substantially entire outer surface defined by the front and rear waist region of the one diaper is in contact with the corresponding entire outer surface of the other diaper. When it is desired to pick up one of these diapers, the upper wall of the package may be broken and one diaper may be finger gripped and drawn out from the package toward above the package.

The diapers disclosed in the above-cited Publications have been sold per package in which a plurality of the diapers are packed. The diapers are closely packed in the package so as to be compressed together in back-and-forth direction between the opposed side walls of the package. Within the package, the row formed by these diapers is compressed inward from longitudinally opposite ends of this row under a pressure in a range of about 15 to 70 N. Therefore, a predetermined force is required to pick the individual diaper out from the row. As has previously been described, the substantially entire surface of the diaper defined by the outer surfaces in its front and waist regions and crotch region is in close contact with the corresponding outer surface of the adjacent diaper. This means that a high frictional force between each pair of the diapers adjacent to each other exerted over the large contact area intends to prevent the individual diaper from being smoothly drawn out from the row. Specifically, a force in a range of 20 to 22 N is required to pick the individual diaper out from the complete row.

It is an object of the present invention to provide a package adapted to pack a plurality of diapers allowing the individual diaper to be smoothly drawn out from the row of these packages.

### DISCLOSURE OF THE INVENTION

According to the present invention, there is provided a package of disposable diapers comprising: a bag made of flexible sheet including a pair of side walls; and a plurality of disposable diapers having a waist-hole, a pair of leg-holes, front and rear waist regions opposed to each other and a crotch region extending between the front and rear waist regions wherein each of the diapers includes a liquid-absorbent core extending between the front and rear waist regions, the diapers being packed within the bag.

In the package, each of the diapers has waist-lateral portions and an intermediate portion extending between the waist-lateral portions, the waist-lateral portions being folded inward between themselves together with lateral portions of the core toward a longitudinal center line bisecting a width dimension defined between edges of the waist-lateral portions; and the diapers are arranged in one direction to form a row between the pair of side walls with the waist middle portion of each of the diapers in close contact one with another and in a state of compression.

According to one preferred embodiment, leg-surrounding lateral portions forming the leg-holes are also folded inward together with the lateral portions of the core extending in the crotch region toward the longitudinal center line.

A pressure in a range of 10 to 75 N is exerted inward upon the row of the diapers in the one direction between the side walls of the bag opposed to each other so that a force of 18 N or lower is enough to draw one of the diapers out from the row.

### BRIEF DESCRIPTION OF THE DRAWINGS

Fig. 1 is a partially cutaway perspective view showing a bag constituting a package of disposable diapers;
Fig. 2 is a perspective view illustrating a manner in which the individual diaper is drawn out from the bag;
Fig. 3 is a perspective view showing the diaper having been drawn out from the bag;
Fig. 4 is a plan view showing a developed planar shape of the diaper illustrated in Fig. 3;
Fig. 5 is a perspective view illustrating alternative manner in which the individual diaper is folded in order to be packed into the package;
Fig. 6 is a plan view showing a developed planar shape of the diaper illustrated in Fig. 5; and
Fig. 7 is a perspective view illustrating a manner in which the diaper folded in the manner illustrated in Fig. 5 is drawn out from the package.

### DETAILED DESCRIPTION OF THE PREFERRED EMBODIMENTS

Details of the package of disposable diapers according to the present invention will be more fully understood from the description given hereunder with reference to the accompanying drawings.

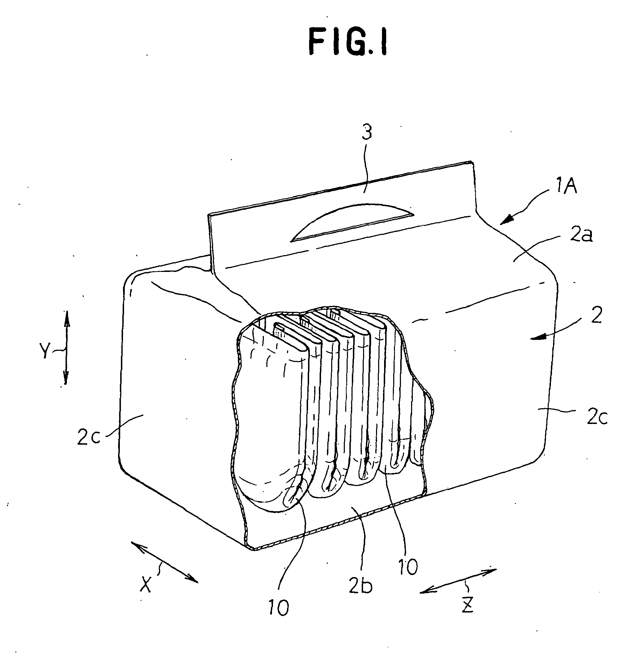

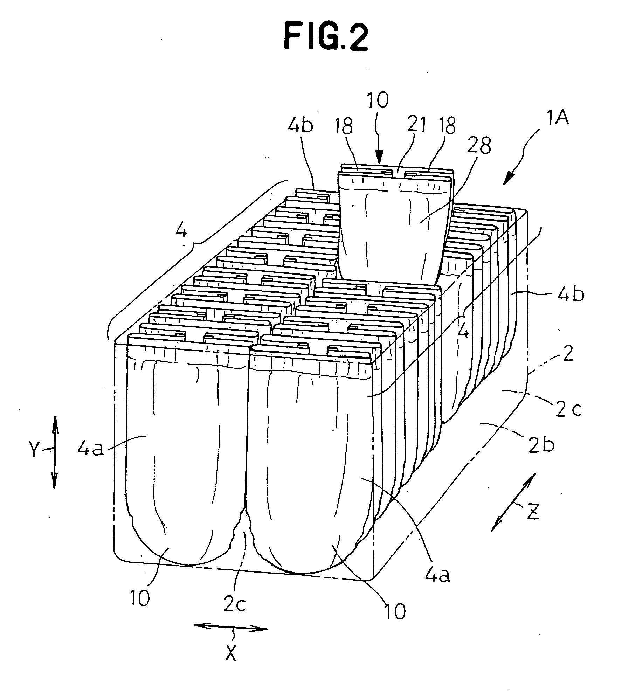

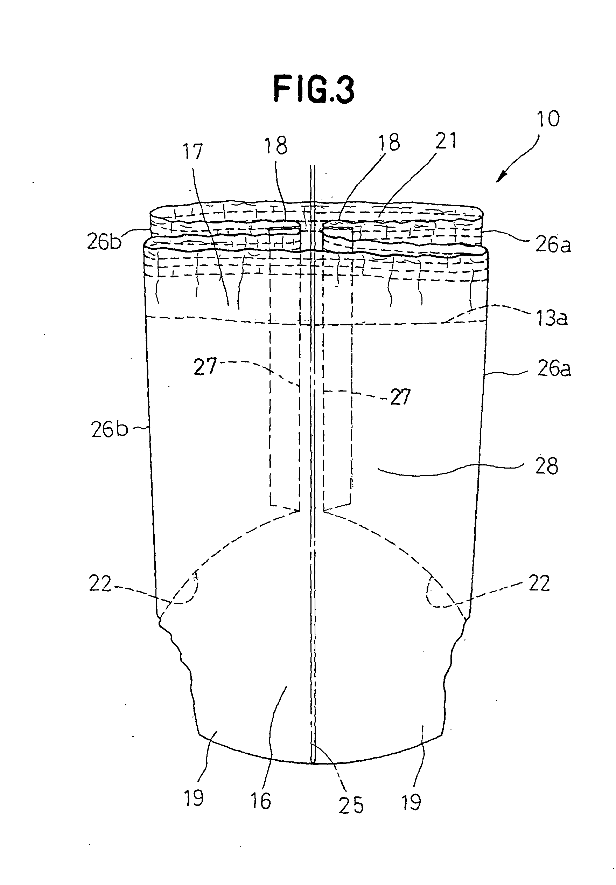

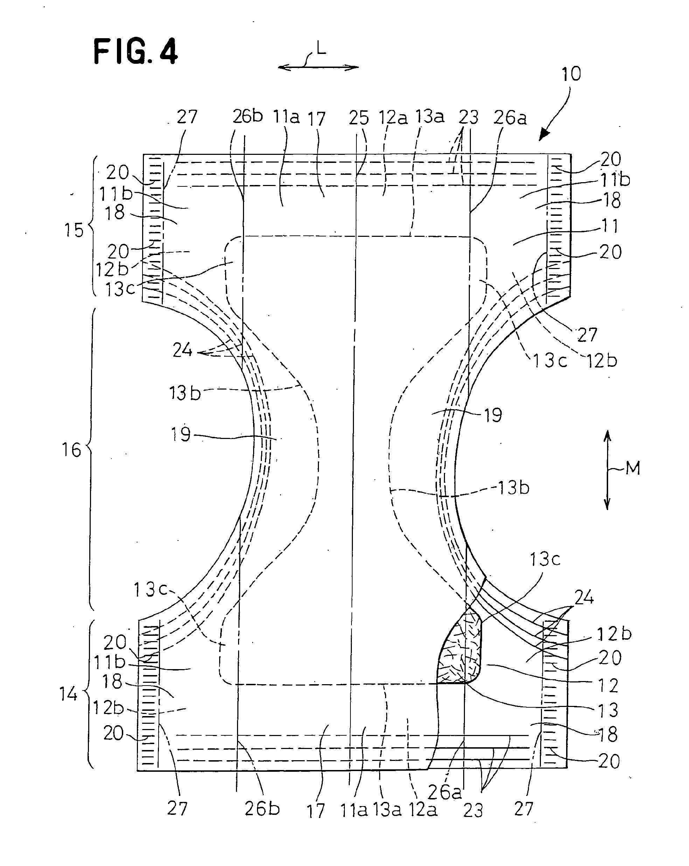

Fig. 1 is a partially cutaway perspective view showing a bag 2 constituting a package 1A of disposable diapers, Fig. 2 is a perspective view illustrating a manner in which the individual diaper 10 is drawn out from the bag 2, Fig. 3 is a perspective view showing the diaper 10 having been drawn out from the bag 2 and Fig. 4 is a developed plan view showing the diaper 10 having its front and rear waist regions 14, 15 disconnected from each other. In Figs. 1 and 2, a transverse direction is indicated by an arrow X, a vertical direction is indicated by an arrow Y and a back-and-forth direction is indicated by an arrow Z. In Fig. 4, a width direction is indicated by an arrow L and a longitudinal direction is indicated by an arrow M.

Within the bag 2 constituting the package 1A, a plurality of flatly folded diapers 10 are arranged in close contact one with another under compression in the back-and-forth direction so that the diapers 10 may be packed as efficiently as possible. The bag 2 is formed by a flexible plastic sheet and has a hexahedral shape which is relatively long in the back-and-forth and defined by top and bottom walls 2a, 2b and four side walls 2c. The top wall 2a of the bag 2 is integrally formed with a handgrip 3. Within the bag 2, eight diapers 10 are arranged in close contact one with another in the back-and-forth direction (one direction) between a pair of opposite side walls 2C, 2C of the bag 2 to form a row 4 and a pair of such rows 4 are arranged side by side. Thus a total of sixteen diapers 10 are packed in the bag 2.

As shown in Figs. 3 and 4, the diaper 10 comprises a liquid-pervious topsheet 11 facing a wearer's body, a liquid-impervious backsheet 12 facing away from the wearer's body and a liquid-absorbent core 13 interposed between the top- and backsheets 11, 12 and secured to an inner surface of at least one of these sheets 11, 12. The diaper 10 defines front and rear waist regions 14, 15 opposed to each other and a crotch region 16 extending between these waist regions 14, 15. The core 13 extends over the crotch region 16 further into the front and rear waist regions 14, 15.

The diaper 10 has waist-surrounding end portions 17 lying outside longitudinally opposite ends 13a of the core 13 and respectively extending in the front and rear waist regions 14, 15 in the width direction, waist-lateral portions 18 lying outside side edges 13c of the core 13 and respectively extending in the front and rear waist regions 14, 15 in the longitudinal direction, and leg-surrounding lateral portions 19 lying outside side edges 13b of the core 13 and extending in the crotch region 16 in a leg-surrounding direction.

The waist-lateral portions 18 in the front waist region 14 and the waist-lateral portions 18 in the rear waist region 15 are overlaid and joined together in the vicinity of respective edges of these lateral portions 18 by a plurality of heat-welding lines 20 arranged intermittently in the longitudinal direction. The diaper 10 is formed with a waist-hole 21 and a pair of leg-holes 22. The leg-surrounding lateral portions 19 in the crotch region 16 describe circular arcs which are respectively convex inward in the width direction of the diaper 10. The diaper 10 has a substantially hourglass-like developed planar shape.

The diaper 10 is formed in the waist-lateral portions 18 with fold-guiding lines 26a, 26b extending in the longitudinal direction as indicated by chain double-dashed lines in Fig. 4. These fold-guiding lines 26a, 26b extend parallel to a longitudinal center line 25 bisecting a width dimension between the edges of the respective lateral portions 18 so as to extend in the respective side edges 13c of the core 13. In the crotch region 16, the diaper 10 is folded back in two in the longitudinal direction with the topsheet 11 kept in contact with itself. The waist-lateral portions 18 also are folded inward together with the lateral portions 13c of the core 13 toward the longitudinal center line 25 along the fold-guiding lines 26a, 26b as well as along imaginary fold-guiding lines 27.

With the lateral portions 18 folded inward, an intermediate portion 28 of the diaper 10 between the waist-lateral portions 18 is exposed out and has a substantially rectangular shape as viewed from the sides of the front and rear waist regions 14, 15 of the diaper 10. In the diaper 10 folded in the manner as has been described above, the diaper 10 has a substantially uniform thickness over the entire extent defined from the waist-surrounding end portion 17 to the crotch region 16. Consequently, the diaper 10 folded in this manner becomes significantly compact compared to before the diaper 10 is folded. In the diaper 10 after folded, the fold-guiding lines 26a, 26b in the front and rear waist regions 14, 15 intersect peripheral edges of the respective leg-holes 22. Consequently, the leg-surrounding lateral portions 19 forming the respective leg-holes 22 are partially folded inward.

Longitudinally opposite end portions 11a, 12a of the top- and backsheets 11, 12 extending outward beyond the longitudinally opposite ends 13a of the core 13 are overlaid together along the waist-surrounding end portion 17 of the diaper 10 and these top- and backsheets 11, 12 have respective inner surfaces permanently bonded together along these end portions 11a, 12a. Transversely opposite side portions 11b, 12b of the top- and backsheets 11, 12 extending outward beyond the side edges 13c of the core 13 are overlaid together along the waist-lateral portions 18 and the leg-surrounding lateral portions 19 and these top- and backsheets 11, 12 have respective inner surfaces permanently bonded together along these side portions 11b, 12b.

The waist-surrounding end portions 17 of the diaper 10 are provided with waist-surrounding elastic members 23. These elastic members 23 are interposed between the top- and backsheets 11, 12 and bonded in a stretched state to inner surfaces of the sheets 11, 12 along the end portions 11a, 12a thereof. The leg-surrounding lateral portions 19 of the diaper 10 are provided with leg-surrounding elastic members 24. These elastic members 24 also are interposed between the top- and backsheets 11, 12 and bonded in a stretched state to inner surface of the sheets 11, 12 along the side portions 11b, 12b thereof.

Each of the diapers 10 packed within the bag 2 has its waist-hole 21 facing the top wall 2a of the bag 2 and its crotch region 16 lying on the bottom wall 2b of the bag 2. Within the bag 2, the diapers 10 are closely packed in a state of compression in the back-and-forth direction with the intermediate portions 28 of the diapers 10 between the waist-lateral portions 18 held in contact one with another. In the package 1B, a pressure in a range of 10 to 75 N (preferably in a range of 20 to 45 N) is exerted upon the row 4 of diapers 10 inward from longitudinally opposite ends 4a, 4b of the row 4 in the back-and-forth direction (in one direction) between the longitudinally opposite side walls 2C, 2C of the bag 2.

According to the package 1A, each of the diapers 10 to be packed into the bag 2 is compactly folded so that the diaper 10 may have a substantially rectangular shape as viewed in the back-and-forth direction and have a thickness substantially uniform in the back-and forth direction. Such manner of folding ensures that no void space is left between each pair of the diapers 10 adjacent to each other when a plurality of diapers 10 are packed into the bag 2 and correspondingly the diapers 10 can be efficiently packed into the bag 2. Compactly folding of the individual diapers 10 is effective also to reduce an area over which each pair of the diapers adjacent to each other come in contact with each other and thereby to alleviate a frictional force (frictional resistance) generated between the diapers adjacent to each other.

To take out a target diaper 10 from the bag 2, after the top wall 2a of the bag 2 has been broken, this diaper 10 may be finger-gripped and drawn upward from the row 4, as illustrated in Fig. 2. In the case of the package 1A shown in Fig. 2, the diapers 10 are put in the bag 2 with the crotch regions 16 thereof facing downward and therefore the diaper 10 may be drawn out toward above the bag 2 with the waist-surrounding end 17 of this diaper 10 gripped by the fingers. Though not shown, in the case of the package 1A in which the diapers 10 are packed with the crotch regions 16 thereof facing upward, the diaper 10 may be drawn out toward above the bag 2 with the crotch region 16 of this diaper 10. Alternatively, after one of the side walls 2c has been broken, the target diaper 10 selected from the associated row 4 may be gripped by the fingers and laterally drawn out from the bag 2.

If the pressure exerted inward upon each of the rows 4 from the longitudinally opposite ends 4a, 4b of this row 4 is lower than 10 N, the row 4 is apt to lean forward or rearward within the package 1A and, when a plurality of the packages 1A are stacked in the vertical direction, such stack of the packages 1A may unintentionally collapse. The pressure exceeding 75 N will make it difficult to smoothly draw the diaper 10 out from the associated row 4.

A pressure exerted inward in the back-and-forth direction upon the row of the diapers 10 from the longitudinal opposite ends 4a, 4b of the row 4 was measured in a manner as follows:
(1) Eight diapers 10 with the waist-lateral portions 18 folded back together with the side edges 13c of the core 13 as seen in Fig. 3 were arranged in the back-and-forth direction within the bag 2 to form the row 4 and left for 24 hours without subjecting the row 4 to any pressure exerted inward from the longitudinally opposite ends 4a, 4b of the row 4 in the back-and-forth direction. In the row 4, the intermediate portions 28 of the diapers 10 exposed in the back-and-forth direction were in contact one with another. The row 4 free from any pressure exerted inward thereupon in the back-and-forth direction had a dimension in the back-and-forth direction larger than a dimension of the bag 2 in the back-and-forth direction;
(2) After left for 24 hours, a pair of plates were placed at the longitudinally opposite ends 4a, 4b of the row 4, respectively, so as to sandwich the row 4. Then these plates were moved inward to compress the row 4 in the back-and-forth direction until the dimension of the row 4 in the back-and-forth direction exactly coincides with the dimension of the bag 2 in the back-and-forth direction;
(3) A pressure exerted upon the plates when the dimension of the row 4 in the back-and-forth direction exactly coincides with the dimension of the bag 2 in the back-and-forth direction was measured by a pressure tester; and
(4) As the pressure tester, INSTRON 5564 (supplied from INSTRON CORP.) was used. A compression rate of this pressure tester was 10 mm/min. As the plates, acryl plates each having a surface area larger than the exposed area of the front and rear waist regions 14, 15 of the diaper 10 so that the plates may compress the entire exposed area of these front and rear waist regions 14, 15. It should be understood here that the dimension of the row 4 in the back-and-forth dimension when it exactly coincides with the dimension of the bag 2 in the back-and forth direction refers to the dimension defined between the pair of acryl plates.

In the case of this package 1A, a force of 18 N or lower is enough to draw one of the diapers 10 out from the row 4 and a force in a range of 15 to 18 N is enough to draw the first diaper 10 out from the row 4. If a force higher than 18 N is necessary to draw one of the diapers 10 out from the row 4, the diaper 10 could not be smoothly drawn out from the bag 2 unless the diaper is drawn with a considerably high force. In addition to such inconvenience, there is a possibility that one or two diapers 10 adjacent to the target diaper 10 might be drawn out from the row 4 as the target diaper 10 is drawn out from the row 4.

A force necessary to the diaper 10 out from the row 4 was measured in a manner as follows:
(1) Eight diapers 10 with the waist-lateral portions 18 folded back together with the side edges 13c of the core 13 as seen in Fig. 3 were arranged in the back-and-forth direction within the bag 2. Within the bag 2, the intermediate portions 28 of the diapers 10 exposed in the back-and-forth direction were in contact one with another to form the row 4, as illustrated in Figs. 1 and 2. Within the bag 2, a pressure in a range of 10 to 75 N was exerted inward upon the row 4 in the back-and-forth direction from the longitudinally opposite ends 4a, 4b of the row 4;
(2) A single diaper 10 was selected from the row 4 and the waist-surrounding end portion 17 was gripped by a chuck of a tensile tester;
(3) The diaper 10 gripped by the chuck was drawn out from the row 4 toward above the bag 2 by means of the tensile tester and a force required to draw the diaper 10 out from the row 4 was measured by the tensile tester; and
(4) As the tensile tester, INSTRON 5564 (supplied from INSTRON CORP.) was used. A tensile rate was 500 mm/min. It should be understood that the maximum value obtained under the measurement condition as set forth above was defined as the force necessary to draw the diaper 10 out from the row 4.

According to the package 1A, a plurality of diapers 10 have been compactly folded so that an area over which each pair of the diapers adjacent to each other come in contact with each other may be reduced and correspondingly a frictional force (frictional resistance) generated between the diapers adjacent to each other may be alleviated compared to the case in which a plurality of diapers 10 are packed in the bag 2 without being folded. Consequently, a force of 18 N or lower is enough to draw the diaper 10, particularly, the first diaper 10 out from the complete row 4 of the diapers 10 regardless of the fact that this complete row 4 is under a pressure in a range of 10 to 75 N exerted inward thereupon in one direction from the longitudinally opposite ends of the row 4.

While the bag 2 is illustrated to contain therein sixteen diapers 10 in two rows 4, the number of diapers 10 to be packed in the bag 2 is not limited to sixteen but optional so far as a plurality of diapers 10 (specifically eight or more diapers 10) are arranged in one direction to from the row 4.

While the force necessary to draw the diaper 10 out from the row 4 has been described above with respect to the case in which the diaper 10 gripped by the chuck of the tensile tester is drawn out from the row 4 toward above the bag 2 and this force is measured by the tensile tester, it is possible also to draw the diaper 10 gripped by the chuck of the tensile tester out laterally from the row 4 and to measure this force by the tensile tester. In this case also, the force required to draw one of the diapers 10 out from the row 4 of the diapers 10 is 18 N or lower and obviously the force required to draw the first diaper 10 out from the row 4 is in a range of 18 to 15 N.

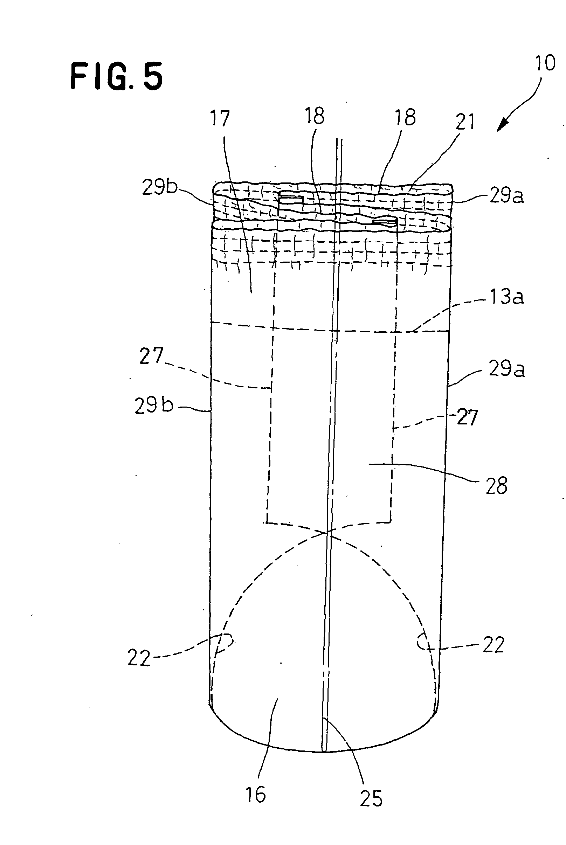

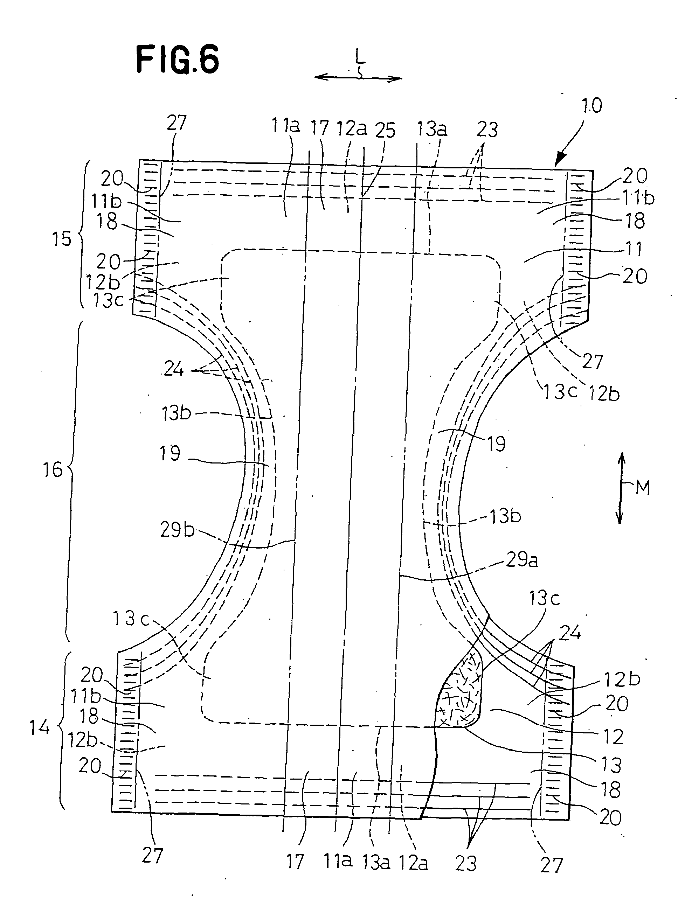

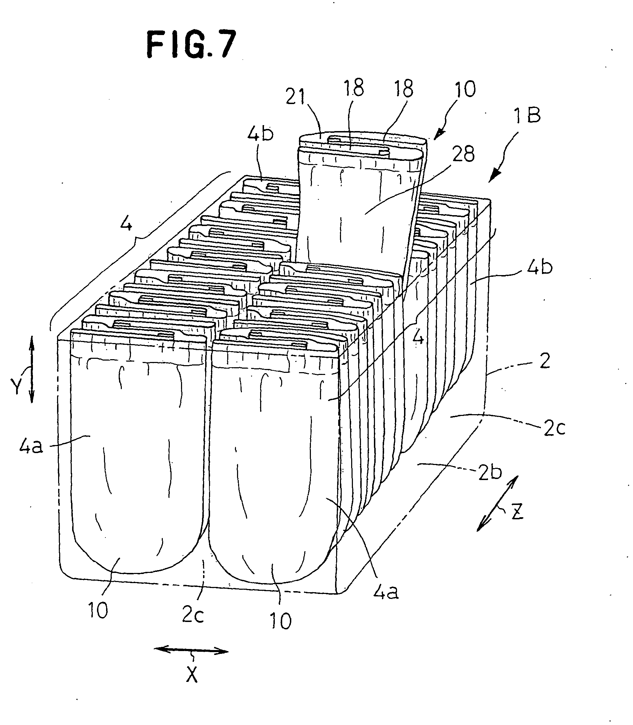

Fig. 5 is a perspective view illustrating alternative manner in which the individual diaper 10 is folded in order to be packed in the package 1B, Fig. 6 is a plan view showing a developed planar shape of the diaper 10 illustrated in Fig. 5 and Fig. 7 is a perspective view illustrating a manner in which the diaper 10 folded in the manner illustrated in Fig. 5 is drawn out from the bag 2.

The diaper 10 is formed in the waist-lateral portions 18 with fold-guiding lines 29a, 29b extending in the longitudinal direction as indicated by chain double-dashed lines. These fold-guiding lines 29a, 29b extend parallel to a longitudinal center line 25 so as to extend in the respective side edges 13c of the core 13 in the front and rear waist regions 14, 15 as well as parts of the side edges 13b of the core 13 in the crotch region 16. In the waist-lateral portions 18 and the crotch region 16, the diaper 10 is folded back along these fold-guiding lines 29a, 29b. In this manner, the waist's side edges 18 are folded inward together with the side edges 13c of the core 13 in the front and rear waist regions 14, 15 toward the longitudinal center line 25. Simultaneously, the leg-surrounding lateral portions 19 destined to form the leg-holes 22 are also folded inward together with the side edges 13b of the core 13 in the crotch region 16.

In the case of this diaper 10, parts of the respective waist-lateral portions 18 in the vicinity of the edges thereof extend beyond the longitudinal center line 25 so as to occupy positions opposed to each other about the longitudinal center line 25 as the waist-lateral portions 18 are folded inward along the fold-guiding lines 29a, 29b. Consequently, the waist-lateral portions 18 overlap each other in a thickness direction of the diaper 10 and the leg-surrounding lateral portions 19 forming the leg-holes 22 are folded along the fold-guiding lines 29a, 29b inward together with the side edges 13b of the core 13 in the crotch region 16. In this way, the intermediate portions 28 of the front and rear waist regions 14, 15 between the waist-lateral portions 28 and the leg-surrounding lateral portions 19 are exposed outward. Therefore, the exposed area of the diaper 10 can be further reduced and the diaper 10 can be made further compact.

In the package 1B, each of the diapers 10 packed within the bag 2 has its waist-hole 21 facing the top wall 2a of the bag 2 and its crotch region 16 lying on the bottom wall 2b of the package 1B. Within the bag 2, the diapers 10 are closely packed in a state of compression in the back-and-forth direction with the intermediate portions 28 of the diapers 10 between the waist-lateral portions 18 and the leg-surrounding lateral portions 19 held in contact one with another. In this package 1B, a pressure in a range of 10 to 75 N (preferably in a range of 20 to 45 N) is exerted upon the row 4 of diapers 10 inward from longitudinally opposite ends 4a, 4b of the row 4 in the back-and-forth direction (in one direction) between the longitudinally opposite side walls 2C, 2C of the bag 2.

Procedure to take out a target diaper 10 from the bag 2 is same as in the case of the bag 2. In the case of the package 1B shown in Fig. 7, a force of 18 N or lower is enough to draw one of the diapers 10 out from the row 4 and a force in a range of 15 to 18 N is enough to draw the first diaper 10 out from the row 4. If a force higher than 18 N is necessary to draw one of the diapers 10 out from the row 4, the diaper 10 could not be smoothly drawn out from the bag 2 unless the diaper 10 is drawn with a considerably high force. In addition to such inconvenience, there is a possibility that one or two diapers 10 adjacent to the target diaper 10 might be drawn out from the row 4 as the target diaper 10 is drawn out from the row 4.

A pressure exerted inward upon the row 4 in the back-and-forth direction from the longitudinally opposite ends 4a, 4b of the row 4 and a force required to draw one of the diapers 10 out from the row 4 in the package 1B of Fig. 7 were measured by the same methods as in the case illustrated in Fig. 1.

Compared to the package 1A of Fig. 2, the package 1B further facilitates the individual diaper 10 to be drawn out from the bag 2 because the area over which each pair of the diapers 10 adjacent to each other come in contact with each other can be further reduced and a frictional force (frictional resistance) generated between the diapers adjacent to each other can be further alleviated.

A stock material for the topsheet 11 may be selected from the group consisting of a hydrophilic fibrous nonwoven fabric, a hydrophobic fibrous nonwoven fabric having a plurality of apertures and a plastic film having a plurality of fine perforations. A stock material for the backsheet 12 may be selected from the group consisting of a hydrophobic fibrous nonwoven fabric, a breathable but liquid-impervious plastic film, composite nonwoven fabric comprising two or more hydrophobic fibrous nonwoven fabric layers laminated one upon another and a composite sheet comprising a hydrophobic fibrous nonwoven fabric and a breathable but liquid-impervious plastic film laminated upon each other. It is also possible to use, as a stock material for the backsheet 12, a composite nonwoven fabric comprising a melt blown fibrous nonwoven fabric having a high water-resistance interposed between two layers of spun bond fibrous nonwoven fabric being high in strength as well as in flexibility.

The nonwoven fabric may be selected from the group consisting of those obtained by spun lace-, needle punch-, melt blown-, thermal bond-, spun bond-, chemical bond- and air-through-processes. The component fiber of nonwoven fabric may be selected from the group consisting of polyolefin-, polyester- and polyamide-based fibers, core-sheath type or side-by-side conjugated fibers of polyethylene/polypropylene or polyethylene/polyester.

The core 13 is a mixture of fluff pulp and super-absorbent polymer particles or a mixture of fluff pulp, super-absorbent polymer particles and thermoplastic synthetic resin fibers, in both cases, compressed to a given thickness. Preferably, the core 13 is entirely wrapped with a liquid-pervious sheet such as a tissue paper or a hydrophilic fibrous nonwoven fabric to prevent the core 13 from getting out of its initial shape and to prevent polymer particles from falling off. The polymer particles may be selected from the group consisting of those of starch-based polymer, cellulose-based polymer and synthetic polymer.

Bonding the top- and backsheets 11, 12, bonding the core 13 to these sheets 11, 12 and bonding the elastic members 23, 24 to these sheets 11, 12 may be carried out using a hot melt adhesive or various thermal welding techniques such as heat-sealing and ultrasonic sealing.

The package of disposable diapers according to the present invention includes within a bag a plurality of diapers each having been compactly folded so that an area over which each pair of the diapers adjacent to each other come in contact with each other may be reduced and correspondingly a frictional force (frictional resistance) generated between the diapers adjacent to each other may be alleviated compared to the case in which a plurality of diapers are put in the bag without being folded. Consequently, a force of 18 N or lower is enough to draw the diaper, particularly, the first diaper out from the complete row of the diapers regardless of the fact that this complete row is under a pressure in a range of 10 to 75 N exerted inward thereupon in one direction from the longitudinally opposite ends of the row.

The package of disposable diapers according to the present invention also includes within a bag a plurality of diapers in which each of the diapers has the leg-surrounding lateral portions forming the leg-holes folded inward together with the opposite side portions of the core in the crotch region toward the longitudinal center line and the intermediate portions of the respective diapers except the waist-lateral portions and the leg-surrounding lateral portions are in close contact one with another. In this way, the diapers can be made further compact and the area of the diapers exposed outward can be further reduced. Correspondingly the frictional force (frictional resistance) generated between each pair of the diapers adjacent to each other in the row can be further alleviated.

## Claims

1. A package of disposable diapers comprising:
a bag made of flexible sheet including a pair of side walls; and
a plurality of disposable diapers having a waist-hole, a pair of leg-holes, front and rear waist regions opposed to each other and a crotch region extending between said front and rear waist regions wherein each of said diapers includes a liquid-absorbent core extending between said front and rear waist regions, said diapers being packed within said bag; wherein:
each of said diapers has waist-lateral portions and an intermediate portion extending between said waist-lateral portions, said waist-lateral portions being folded inward between themselves together with lateral portions of said core toward a longitudinal center line bisecting a width dimension defined between edges of said waist-lateral portions; and
said diapers are arranged in one direction to form a row between said pair of side walls with said waist middle portion of each of said diapers in close contact one with another and in a state of compression.

2. The package according to Claim 1, wherein leg-surrounding lateral portions forming said leg-holes are also folded inward together with said lateral portions of said core extending in said crotch region toward said longitudinal center line.

3. The package according to Claim 1 or 2, wherein a pressure in a range of 10 to 75 N is exerted inward upon said row of said diapers in said one direction between said side walls of said bag opposed to each other so that a force of 18 N or lower is enough to draw one of said diapers out from said row.
